# FASCICULE DE BREVET EUROPEEN

(11) **EP 1 147 077 B1**
(45) Date de publication et mention de la délivrance du brevet: **23.07.2008**
(21) Numéro de dépôt: 00900534.9
(22) Date de dépôt: 07.01.2000
(51) Int. Cl.: C07C 257/06, C07C 245/04, C07C 257/14

(54) **PROCEDE DE PREPARATION D'AZOIMINOETHERS**
VERFAHREN ZUR HERSTELLUNG VON AZIMINOETHERN
METHOD FOR PREPARING AZOIMINOETHERS

(30) Priorité: 15.01.1999 FR 9900391
(43) Date de publication de la demande: 24.10.2001
(73) Titulaire: ARKEMA FRANCE, 92700 Colombes (FR)
(72) Inventeur: BOURDAUDUCQ, Paul, F-69630 Chaponost (FR)
(74) Mandataire: Hirsch & Associés
(86) Numéro de dépôt international: PCT/FR2000/000022
(87) Numéro de publication internationale: WO 2000/042000

(56) Documents cités:
- EP-A- 0 080 275
- EP-A- 0 230 586
- CHEMICAL ABSTRACTS, vol. 109, no. 7, 15 août 1988 (1988-08-15) Columbus, Ohio, US; abstract no. 54300, KELLY, DAVID P. ET AL: "The cross-reaction between 1-(methoxycarbonyl)- and 1-(butoxycarbonyl)-1-methylethyl: simultaneous generation of unlike radicals from an unsymmetrical azo precursor" XP002122204 & AUST. J. CHEM. (1987), 40(10), 1631-9, 1987,

## Description

La présente invention a pour objet un procédé de préparation d'azoiminoéthers (sous forme de chlorhydrate) et leur hydrolyse en esters d'acides azocarboxyliques, ces esters ainsi préparés étant utiles en tant qu'initiateur de radicaux libres dans les réactions de polymérisation.

La préparation des esters d'acides azocarboxyliques est classiquement mise en oeuvre par un procédé en deux étapes comprenant une première étape de conversion de l'azonitrile, par réaction avec un alcool, en présence de HCl, selon la réaction de Pinner, conduisant au chlorhydrate d'azoiminoéther correspondant et une seconde étape d'hydrolyse en présence d'eau du chlorhydrate ainsi obtenu.

Ce procédé présente plusieurs inconvénients qui le rendent totalement inadapté pour une production à l'échelle industrielle. Il est en effet trop coûteux, difficilement contrôlable, la purification du produit final est difficile et il nécessite un large excès d'alcool. De plus il conduit à un rendement insuffisant et à un produit final d'une pureté non satisfaisante. Un tel procédé est décrit par exemple par G.A. Mortimer dans le Journal de chimie organique page 1632-33 (1965) pour la préparation de l'azobisisobutyrate de diméthyle, utilisé comme intermédiaire dans la synthèse d'un initiateur de polymérisation: le diacétate d'azobisisobutanol.

Pour résoudre certains de ces problèmes et permettre ainsi une production à l'échelle industrielle du procédé de préparation d'esters d'acides azocarboxyliques plusieurs solutions ont été proposées.

Un procédé tel que décrit ci-dessous conduisant à une augmentation du rendement et simultanément à une réduction importante de la durée de la réaction ainsi qu'à une séparation des phases bien meilleure et beaucoup plus rapide entre l'ester azodiisobutyrique et la phase aqueuse est décrit dans DE 2254572. Selon ce brevet, de telles améliorations peuvent être obtenues en réalisant la conversion de Pinner en présence d'un éther cyclique soluble dans l'eau et/ou, d'un diol de bas PM soluble dans l'eau et/ou d'un étherdiol ou d'un polyétherdiol linéaire de PM faible allant jusqu'à 1800, en une quantité de 0,001 à 11,0% en poids ramenée à l'alcool aliphatique en C1-C6.

EP80275 divulgue que le 2,2'-azobis(2-méthyl-propionitrile) ainsi que les composés apparentés peuvent être convertis avec un excellent rendement en utilisant la réaction de Pinner avec seulement la quantité stoechiométrique de l'alcool, si la réaction est mise en oeuvre en présence d'un composé contenant un groupe éther. Il est décrit qu'une conversion plus rapide (et non un rendement et/ou une sélectivité plus élevée) peut être obtenue en augmentant la concentration en HCl dans le mélange réactionnel. La présence d'éther pose cependant des problèmes, notamment au niveau de la séparation et du traitement ultérieur.

EP230586 a mis en évidence que la préparation des azoiminoéthers peut être réalisée en une seule étape dans un seul récipient selon une réaction très facilement contrôlable au cours de laquelle l'halogéno-oxydation de l'hydrazonitrile et l'iminoéthérification de l'azonitrile ont lieu. Le procédé de préparation selon EP230586 comprend donc la réaction d'un hydrazonitrile avec du chlore en présence d'un alcool capable de convertir le groupe cyano en un groupe iminoéther en présence de HCl (qui est formé in-situ). Cette réaction est mise en oeuvre dans un système non-aqueux, en présence d'un solvant utilisé dans l'halogéno-oxydation et iminoéthérification, tel que les hydrocarbures aromatiques, les hydrocarbures halogénés et certains autres solvants. La quantité d'alcool utilisée varie entre la quantité théorique requise et 1,2 fois cette quantité et la quantité de chlore varie entre la quantité théorique et un léger excès. Le produit final de la réaction correspondant au chlorhydrate d'azoiminoéther peut être converti en un azoester par hydrolyse.

La demande de brevet JP-A-50-105614 décrit une méthode de préparation d'azoiminoéthers à partir d'azonitriles, par action d'un alcool en présence d'acide chlorhydrique à l'état gazeux dans un solvant dans lequel l'acide chlorhydrique gaz a une faible solubilité.

Cependant, aucun des documents ci-dessus ne fournit de procédé qui puisse être utilisé à l'échelle industrielle, avec un rendement élevé et avec une pureté suffisante qui permet de s'affranchir de techniques de séparation lourdes.

La demanderesse a mis en évidence de façon surprenante un nouveau procédé de préparation des sels d'azoiminoéthers et des esters d'acides azocarboxyliques correspondants qui atteint les buts ci-dessus. La présente invention concerne donc un procédé de préparation de sels d'azoiminoéthers par conversion de l'azonitrile selon la réaction de Pinner dans laquelle la réaction est mise en oeuvre dans un solvant aromatique et en présence d'un large excès d'HCl.

La présente invention a donc pour objet un procédé de préparation d'un chlorhydrate d'azoiminoéther comprenant la réaction d'un azonitrile entre 15 et 25°C, pendant une durée de 8 à 24 heures, avec un alcool choisi parmi l'éthanol ou un alcool supérieur ou un mélange d'alcools et de l'acide chlorhydrique dans un solvant aromatique, dans lequel le rapport molaire R = HCl/azonitrile est compris entre 4 et 6.

Selon un mode de réalisation particulier, l'azonitrile est formé in situ par réaction de l'hydrazonitrile correspondant avec du chlore.

Selon un autre mode de réalisation, le solvant est sélectionné dans le groupe consistant en toluène, chlorobenzène, xylène, benzène; le chlorobenzène étant utilisé de préférence lorsque l'azonitrile est formé *in situ*.

Selon encore un autre de mode de réalisation, l'alcool est l'éthanol.

Selon un autre mode de réalisation, l'alcool utilisé est constitué d'un mélange d'alcools, en particulier un mélange comprenant du méthanol, ou du méthanol et de l'éthanol.

Selon un mode de réalisation du procédé de préparation selon l'invention, le chlorhydrate d'azoiminoéther répond à la formule (II) 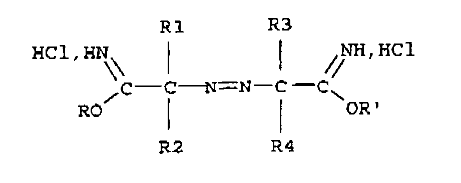 dans laquelle :
R1, R2, R3 et R4, identiques ou différents, sont sélectionnés indépendamment dans le groupe consistant en : alkyles linéaires ou ramifiés en C1-C9 (de préférence C1-C4) non substitués, ou substitués par un ou plusieurs substituants sélectionnés parmi les substituants hydroxyle, alkoxy en C1-C6, halogène;
cycloalkyles en C3-C6, non substitués ou substitués par un ou plusieurs substituants sélectionnés parmi alkyle en C1-C6, alkoxy en C1-C6, hydroxy, halogène;
aralkyles en C7-C12, non substitués ou substitués par un ou plusieurs substituants sélectionnés parmi alkyle en C1-C6, alkoxy en C1-C6, hydroxy, halogène;
aryles en C7-C12 non substitués ou substitués par un ou plusieurs substituants sélectionnés parmi alkyle en C1-C6, alkoxy en C1-C6, hydroxy, halogène;
au moins une des combinaisons R1-R2 et R3-R4 pouvant éventuellement former un cycle aliphatique,
R et R', identiques ou différents, sont sélectionnés indépendamment dans le groupe consistant en radicaux aliphatiques linéaires ou ramifiés en C1-C10, de préférence en C1-C4.

Selon un autre mode de réalisation de la présente invention, dans la formule (II), R et R' sont différents l'un de l'autre et sont sélectionnés parmi les radicaux aliphatiques linéaires en C1-C4,

Selon un mode particulier de réalisation, R1, R2, R3 et R4 sont des groupes alkyles en C1-C4.

L'invention a également pour objet un procédé de préparation d'un ester d'acide azocarboxylique comprenant la synthèse d'un chlorhydrate d'azoiminoéther par le procédé tel que défini ci-dessus et l'hydrolyse en présence d'eau du chlorhydrate d'azoiminoéther ainsi obtenu.

Selon un mode de réalisation, l'hydrolyse est mise en oeuvre par addition successive d'eau au mélange réactionnel ou par coulée du mélange réactionnel dans de l'eau, à une température comprise entre 15°C et 50°C, de préférence d'environ 30°C

Selon un mode spécifique de réalisation du procédé de préparation d'un ester d'acide azocarboxylique, après l'étape de synthèse, le chlorhydrate d'azoiminoéther est filtré, lavé avec un solvant organique puis l'hydrolyse est mise en oeuvre par addition progressive du gâteau de filtration dans de l'eau à une température comprise entre 15°C et 50°C, de préférence entre 25°C et 35°C.

L'invention a en outre pour objet un procédé de préparation d'une composition liquide d'esters d'acides azocarboxyliques comprenant la synthèse du chlorhydrate d'azoiminoéthers par le procédé tel que décrit ci-dessus, l'hydrolyse des sels ainsi obtenus en présence d'eau et l'isolement de la phase organique contenant les esters.

Selon un mode de réalisation particulier de ce procédé de préparation, on fait réagir dans un premier temps l'alcool le plus lourd puis dans un deuxième temps l'alcool le moins lourd.

Selon un mode de réalisation, la composition liquide comprend un premier ester symétrique d'un premier alcool, un second ester symétrique d'un second alcool, et un ester mixte de ces premier et second alcools. Selon ce mode de réalisation, ledit premier ester symétrique est l'ester symétrique méthylique, ledit second ester symétrique est l'ester symétrique éthylique et ledit ester mixte est l'ester méthylique-éthylique.

L'invention a également pour objet un procédé de préparation d'initiateurs de polymérisation comprenant la synthèse d'un ester d'acide azocarboxylique par le procédé de préparation des esters d'acides azocarboxyliques tel que décrit ci-dessus et le cas échéant la transformation de cet ester en initiateur par des procédés connus.

La présente invention a également pour objet un procédé de préparation de dérivé azoguanyl comprenant la synthèse du chlorhydrate d'azoiminoéther correspondant par le procédé tel que décrit ci-dessus et la réaction de ce dernier avec de l'ammoniac ou une amine en présence d'alcool par tout procédé connu approprié à cette fin.

L'invention est maintenant décrite plus en détail dans la description qui suit.

Dans le procédé de préparation du chlorhydrate d'azoiminoéther selon l'invention, l'azonitrile de départ utilisé dans la réaction de conversion de Pinner peut être symétrique ou asymétrique. En tant qu'exemple de tels azonitrile on peut citer les azonitriles répondant à la formule (I) 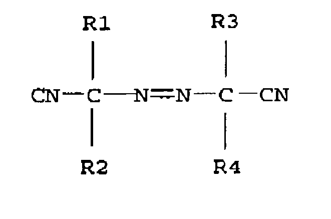 dans laquelle:
R1, R2, R3 et R4, identiques ou différents, sont sélectionnés indépendamment dans le groupe consistant en: alkyles linéaires ou ramifiés en C1-C9 (de préférence C1-C4) non substitués, ou substitués par un ou plusieurs substituants sélectionnés parmi les substituants hydroxyle, alkoxy en C1-C6, halogène;
cycloalkyles en C3-C6, non substitués ou substitués par un ou plusieurs substituants sélectionnés parmi alkyle en C1-C6, alkoxy en C1-C6, hydroxy, halogène;
aralkyles en C7-C12, non substitués ou substitués par un ou plusieurs substituants sélectionnés parmi alkyle en C1-C6, alkoxy en C1-C6, hydroxy, halogène;
aryles en C7-C12 non substitués ou substitués par un ou plusieurs substituants sélectionnés parmi alkyle en C1-C6, alkoxy en C1-C6, hydroxy, halogène;
au moins une des combinaisons R1-R2 et R3-R4 pouvant éventuellement former un cycle aliphatique.

Des exemples concrets de tels azonitriles sont : 2,2'-azobisisobutyronitrile, 2,2'-azobis(a-méthylbutyronitrile), 2,2'-azobis(2,4-diméthylvaléronitrile), 1,1'-azobis(1-cyanocyclohexane).

S'agissant de l'alcool utilisé dans la réaction de conversion de Pinner, on utilise des alcools aliphatiques linéaires ou ramifiés, en C1-C10, de préférence linéaires et de préférence en C1-C4. L'éthanol et/ou le méthanol (dans le cas des mélanges d'alcools) sont particulièrement préférés. L'alcool est utilisé en une quantité égale à la quantité stoechiométrique requise ou en un léger excès par rapport à cette dernière c'est à dire jusqu'à 1,5 fois la valeur théorique. On entend par alcool dans le cadre de la présente invention également les mélanges des alcools tels que définis ci-dessus, de préférence les mélanges contenant au moins du méthanol. Dans un tel cas de figure des mélanges d'esters sont obtenus. Par exemple, si on utilise un mélange méthanol/éthanol en tant que réactif, on obtient un ester de méthyle, un ester d'éthyle et un ester mixte d'éthyle-méthyle. Les mélanges d'alcools incluent également les mélanges de plus de 2 alcools, tels que par exemple un mélange méthanol/éthanol/propanol.

L'acide chlorhydrique est utilisé en large excès par rapport à la quantité stoechiométrique requise. La demanderesse a mis en évidence de façon surprenante d'une part que l'excès de HCl devait être très important (jusqu'à par exemple 3 fois la stoechiométrie) et d'autre part que la quantité d'HCl devant être ajoutée dépend aussi de la nature de l'alcool utilisé dans l'étape de conversion de Pinner. Le rapport molaire R HCl/azonitrile est compris entre 4 et 6 lorsqu'il s'agit de l'éthanol ou d'un alcool supérieur. Dans le cas où l'on utilise des mélanges d'alcools, la valeur de R est la moyenne pondérée des valeurs R en présence pour chaque alcool individuellement. Par exemple dans le cas d'un mélange méthanol/éthanol 50/50 molaire la valeur R du rapport HCl/azonitrile du mélange remplit alors généralement la condition R ≥ 2+3/2=2,5. R est compris entre 4 et 6 pour les alcools supérieurs.

S'agissant du solvant aromatique, on peut utiliser tout solvant aromatique, halogéné ou non halogéné, qui soit suffisamment volatil pour être éliminé à la fin de la réaction par évaporation à une température relativement basse sous pression réduite. Des solvants particulièrement appropriés incluent chlorobenzène, toluène, xylène, benzène.

La réaction de conversion de Pinner est mise en oeuvre à une température en général de 15 à 25°C pendant une durée qui varie selon la nature de l'azonitrile et la température réactionnelle et qui est de l'ordre de 8 à 24H. On réalise en général la réaction de conversion selon l'invention de la manière suivante : on mélange le solvant et l'alcool, et on ajoute au mélange ainsi obtenu l'azonitrile. On introduit ensuite dans le mélange réactionnel -de façon connue- la quantité requise d'acide chlorhydrique anhydre en maintenant la température entre 10 et 40°C, de préférence entre 15 et 25°C. Le procédé peut être réalisé aussi bien sans que sous pression.

Selon un mode spécifique de réalisation l'azonitrile est préparé in situ à partir de l'hydrazonitrile correspondant par réaction avec du chlore et un alcool capable de convertir le groupe cyano en un groupe iminoéther en présence de HCl, comme cela est décrit dans EP230586. Mais contrairement à ce qui est indiqué dans ce document, la réaction doit être mise en oeuvre en présence d'un large excès d'HCl tel qu'il est défini ci-dessus en relation avec la conversion de Pinner. Dans ce cas de figure le rapport R représente (HCl formé *in situ* + HCl ajouté) / hydrazonitrile.

La réaction de l'invention peut être mise en oeuvre pour l'hydrazonitrile sec, mais la demanderesse a mis en évidence qu'il est possible de la réaliser à partir de l'hydrazonitrile humide. Dans ce cas, l'eau est éliminée par dissolution de l'hydrazonitrile humide dans le solvant réactionnel et décantation de la phase aqueuse. Les traces d'eau dissoutes dans le solvant peuvent être éliminées par entraînement azéotropique avant d'ajouter l'alcool et les autres réactifs.

La demanderesse a aussi mis en évidence de plus que la réaction in situ, lorsqu'elle est mise en oeuvre en présence d'un solvant halogéné, particulièrement chloré, préférentiellement du chlorobenzène ne conduit pas à des dérivés chlorés toxiques lors de la préparation des sels d'azoiminoéthers.

Les chlorhydrates d'azoiminoéthers ainsi préparés répondent en général à la formule (II) 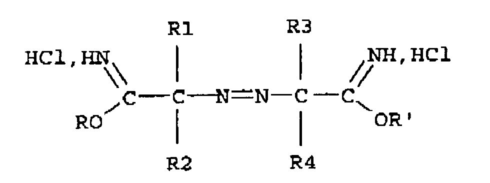 dans laquelle:
dans laquelle:
   R1, R2, R3 et R4 sont tels que définis ci-dessus et
   R et R', identiques ou différents, sont sélectionnés indépendamment dans le groupe consistant en radicaux aliphatiques linéaires ou ramifiés en C1-C10, de préférence en C1-C4 ; R et R' étant de préférence différents.

Les sels d'azoiminoéthers dans lesquels R1, R2, R3 et R4 et R et R' représentent des alkyles en C1 à C4 sont plus particulièrement préférés.

Le chlorhydrate d'azoiminoéther ainsi obtenu peut être utilisé pour la préparation de composés du type azoguanyl par réaction avec l'ammoniac gaz ou une amine primaire ou secondaire en présence d'alcool par tout procédé connu approprié à cette fin.

Le chlorhydrate d'azoiminoéther ainsi obtenu peut être également utilisé pour la préparation d'un ester d'acide azocarboxylique de formule (III) 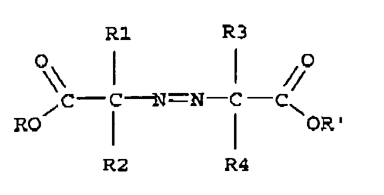 dans laquelle:
R1, R2, R3, R4, R et R' sont tels que définis ci-dessus en relation avec les sels d'azoiminoéthers.

Pour la préparation des esters, à la fin de la conversion de l'azonitrile (fin de réaction que l'on peut déterminer de façon connue par analyse infrarouge de la bande CN), on réalise une hydrolyse après isolement ou non du sel d'azoiminoéther par filtration. A la fin de la réaction, on obtient deux phases limpides. On laisse décanter jusqu'à séparation complète des phases. On élimine la phase aqueuse et la phase organique restante comprenant l'ester d'acide azocarboxylique est concentrée, sous pression réduite pour éliminer le solvant. Une variante de l'isolement du produit final consiste après l'hydrolyse à éliminer le solvant réactionnel par entraînement azéotropique avec l'eau sous pression réduite puis à décanter la phase organique supérieure.

Pour obtenir un produit final d'une pureté satisfaisante, il est important de minimiser la quantité d'azonitrile restant dans le produit final ; les produits de décomposition des azonitriles (en particulier dans le cas du 2,2'-azobisisobutyronitrile, avec la formation de tétraméthylsuccinonitrile) étant assez toxiques. Dans le cas de l'isolement du chlorhydrate d'azoiminoéther par filtration et lavage par un solvant organique (tel que cyclohexane, toluène), une purification est faite, l'azonitrile étant soluble dans ces solvants. L'hydrolyse peut être ensuite réalisée par addition progressive du gâteau de filtration dans de l'eau à une température de 15 à 50°C, de préférence entre 25°C et 35°C.

Par contre, dans le but d'éviter l'étape de filtration d'un produit de faible stabilité, la demanderesse a mis au point l'hydrolyse par ajout progressif d'eau dans la suspension de chlorhydrate d'azoiminoéther dans le solvant réactionnel ou par coulée de cette suspension dans de l'eau en contrôlant la température, par exemple à une valeur comprise entre 15 et 50°C, de préférence entre 25 et 35°C.

La présente invention concerne également un procédé de préparation d'une composition liquide d'esters (mixtes et/ou symétriques) d'acides azocarboxyliques. Ce procédé comprend une première étape de synthèse de sels d'azoiminoéthers, soit par réaction de l'azonitrile, soit par synthèse in situ d'azonitrile à partir de l'hydrazonitrile correspondant, en présence d'un mélange d'alcools comme cela est décrit ci-dessus, puis l'hydrolyse des sels obtenus et la séparation de la phase organique contenant les esters par toute technique classique appropriée. Les compositions liquides ainsi obtenues présentent l'avantage d'être liquide à des températures proches de l'ambiante, et dans certains cas jusqu'à -20°C, faciles à manipuler, ne dégagent pas de poussière, sont non toxiques et non cyanées. Pour la synthèse de ces compositions, les différentes voies décrites en relation avec le procédé de préparation des esters d'acides azocarboxyliques sont applicables (ajout d'eau dans la suspension de chlorhydrate d'azoiminoéther, coulée de cette suspension dans l'eau, ou filtration du chlorhydrate d'azoiminoéther et ajout du gâteau de filtration dans l'eau). Au lieu de partir d'azonitrile on peut partir de l'hydrazonitrile, dans ce cas on utilisera de préférence comme solvant réactionnel le chlorobenzène. Une façon de favoriser la formation de l'ester mixte est de faire réagir dans un premier temps l'alcool plus lourd, puis dans un deuxième temps l'alcool le moins lourd. La demanderesse a aussi mis en évidence que cette synthèse des esters mixtes est aussi dépendante de l'excès d'HCl ajouté, pour un même mélange d'alcools de départ.

La présente invention a également pour objet un procédé de préparation d'initiateurs de polymérisation comprenant la synthèse d'un ester d'acide azocarboxylique par le procédé tel que décrit ci-dessus et le cas échéant la transformation de cet ester en initiateur par des procédés connus. L'invention s'applique aussi à la préparation de tous les composés qui peuvent dériver des esters d'acides azocarboxyliques, tels que : les alcools et les acétates correspondants, les alcanes, les acides et les amides correspondants.

Les sels d'azoiminoéthers mixtes plus particulièrement préférés sont ceux dérivés d'un mélange d'alcools sélectionnés parmi: méthanol, éthanol, n-propanol et n-butanol, de préférence d'un mélange contenant au moins du méthanol.

Les esters d'acides azocarboxyliques obtenus à partir de ces éthers mixtes font également partie de la présente invention.

Les exemples suivants sont donnés à titre purement illustratif et nullement limitatif de l'invention.

### EXEMPLES

### Exemple 1

A un mélange comprenant 600 g de toluène et 76,8 g de méthanol ou 110,4g d'éthanol (2,4 moles), on ajoute 164 g (1 mole) de 2,2'-azobisisobutyronitrile. En refroidissant à 15-20°C, on ajoute de l'acide chlorhydrique gazeux en 4-5 heures. On maintient sous agitation pendant 16 heures à environ 20°C.

On filtre puis on lave le gâteau de filtration avec 2 fois 100 g de cyclohexane. Le gâteau de chlorhydrate d'azoiminoéther est ajouté lentement dans 600 g d'eau à une température d'environ 30°C. Après 1 heure d'agitation à environ 30°C, le mélange réactionnel est refroidit à environ 10°C. Les phases aqueuse et organique sont séparées par décantation de la phase organique et extraction de la phase aqueuse par 100 g de cyclohexane. Les phases organiques ainsi obtenues sont rassemblées et concentrées sous pression réduite à environ 35°C.

Ce procédé est mis en oeuvre pour différentes valeurs du rapport molaire HCl/azonitrile, telles qu'indiquées dans le tableau 1 ci-dessous. Le rendement en azoester obtenu ainsi que la nature de l'alcool sont indiqués dans le tableau 1.

**TABLEAU 1.**

| Alcool | Rapport R: HCl/azonitrile | Rendement en azoester (%) |
|---|---|---|
| CH3OH | 2,0 | 86 |
| | 2,6 | 90,5 |
| | 3,0 | 91,0 |
| C2H5OH | 2,0 | 25,0 |
| | 2,6 | 52,0 |
| | 3,2 | 60,0 |
| | 4,0 | 84,0 |
| | 4,4 | 91,0 |
| | 4,8 | 91,0 |

Ces résultats démontrent clairement que le rapport HCl/azonitrile influence fortement le rendement de la réaction et dépend de la nature de l'alcool.

Ce résultat a également été confirmé en réalisant les exemples de synthèse d'azoiminoéther décrit dans EP230586 en faisant varier la nature de l'alcool dans l'exemple 2 ci-dessous.

### Exemple 2

A un mélange de 640 g de toluène et 76,8 g de méthanol ou 110,4 g d'éthanol (2,4 moles) on ajoute 166g de 2,2'-hydrazobisisobutyronitrile (1 mole). En refroidissant à 15-20°C, on ajoute 74,6g de chlore (1,05 mole, ce qui conduit, par réaction avec une mole d'hydrazonitrile, à la formation in situ de 2,0 moles d'HCl). On agite 5 heures à 25°C puis 15 heures à environ 20°C. Les étapes ultérieures sont mises en oeuvre dans des conditions opératoires identiques à celles décrites dans l'exemple précédent. Le rapport HCl/azonitrile est ici d'environ 2,6, correspondant à la somme de 2,0 moles formées in situ et 0,6 mole ajoutée.

Avec le méthanol le rendement en azoester est de 88%, alors qu'avec l'éthanol le rendement n'est que de 25%.

Les même essais effectués à partir d'hydrazoïque en rajoutant après chloration de l'acide chlorhydrique (0,6 mole dans le cas du méthanol et 2,4 moles dans le cas de l'éthanol, ce qui conduit à des ratios R respectivement d'environ 2,6 et 4,4) ont permis d'obtenir des rendements en azoester de 91 et 90% respectivement. Dans ce cas, après ajout du chlore, on ajoute, à une température de 15-20°C, 21,9 g d'acide chlorhydrique (0,6 mole) dans l'essai au méthanol en environ 1 heure, 87,6 g d'acide chlorhydrique (2,4 mole) dans l'essai à l'éthanol en environ 2 heures. On maintient ensuite sous agitation 15 heures à 20°C. Les étapes ultérieures étant identiques à celles décrites précédemment.

### Exemple 3

Dans 600g de toluène on ajoute 200g de 2,2'-hydrazobisisobutyronitrile humide (1 mole) en chauffant à environ 35°C pour obtenir la dissolution de l'hydrazobisisobutyronitrile. On décante la phase aqueuse inférieure soit 31g. On élimine l'eau dissoute dans le toluène par entraînement azéotropique sous vide. On ajoute 110,4g d'éthanol (2,4 moles) et 0,2g de bromure de sodium utilisé comme catalyseur de chloration. En refroidissant à environ 20°C, on ajoute en 2 heures 74,6g de chlore (1,05 mole, ce qui conduit à la formation in situ de 2,0 moles d'HCl) puis en 3 heures 87,6g d'acide chlorhydrique (2,4 moles). Le ratio R est d'environ 4,4. On maintient le mélange réactionnel sous agitation pendant la durée d'une nuit à 20°C. On coule ensuite le mélange ainsi obtenu dans 600g d'eau préchauffée à environ 25°C sans dépasser 30°C. On agite 1 heure à 30°C. On élimine le toluène par entraînement azéotropique sous vide à une température de 35°C. On décante la phase organique supérieure. Le rendement en azoester est de 91% et la teneur en azonitrile du produit final est de 0,3%.

Des essais effectués dans les mêmes conditions, mais en remplaçant l'éthanol par le méthanol, ont donné avec ajout de 0,6 mole d'acide chlorhydrique (au lieu de 2,4) 0,8% d'azonitrile dans le produit final. Sans ajout d'HCl, il reste 5% d'azonitrile dans le produit final.

### Exemple 4

Dans 750g de chlorobenzène on ajoute 200g de 2,2'-hydrazobisisobutyronitrile humide (1 mole) en chauffant à environ 30°C pour obtenir la dissolution de l'hydrazobisisobutyronitrile. On décante la phase aqueuse supérieure soit 30g. On élimine l'eau dissoute dans le chlorobenzène par entraînement azéotropique sous vide. On ajoute 76,8g de méthanol (2,4 moles) et 0,2g de bromure de sodium. En refroidissant à environ 15°C, on ajoute 74,6g de chlore (1,05 mole, ce qui conduit à la formation in situ de 2,0 moles d'HCl) puis 21,9g d'acide chlorhydrique (0,6 mole). Le mélange réactionnel est agité pendant 18 heures à environ 20°C. Le mélange est ensuite coulé en environ 1 heure dans 600g d'eau, on laisse la température atteindre environ 30°C puis on refroidit pour que la température ne dépasse pas cette valeur. On agite ensuite pendant 1 heure à environ 30°C pour obtenir des phases aqueuse et organique limpides. On élimine le chlorobenzène par entraînement azéotropique sous vide à une température de 35°C. On décante la phase organique supérieure. Le rendement en azoester ainsi obtenu est de 92%.

### Exemple 5

A un mélange de 600g de toluène, 51,2g de méthanol (1,6 mole) et 36,8g d'éthanol (0,8 mole), on ajoute 164g de 2,2'-azobisisobutyronitrile (1 mole). En refroidissant à 15-20°C, on ajoute de l'acide chlorhydrique gazeux en 4-5 heures. Le mélange réactionnel est maintenu sous agitation pendant 16 heures à 20°C. On coule le mélange dans 600g d'eau en 1 heure en laissant monter la température à environ 30°C. Le mélange résultant est agité pendant 1 heure à 30°C pour obtenir des phases aqueuse et organique limpides. Le toluène est éliminé par entraînement azéotropique sous vide à une température d'environ 35°C, puis on décante la phase organique supérieure.

On obtient avec des rendements voisins de 85% des produits limpides à température ambiante, de composition différente suivant la quantité d'acide chlorhydrique utilisée et se figeant à des températures différentes par refroidissement.

**TABLEAU 2**

| Composition, % molaire | R (moles) HCl/azonitrile | | |
|---|---|---|---|
| | 2,6 | 3,3 | 4,3 |
| A | nd | 60,0 | 61,0 |
| B | nd | 25,0 | 30,0 |
| C | nd | 4,0 | 6,0 |
| Divers | nd | 11,0 | 3,0 |
| rapport molaire -COOCH₃/-COOC₂H₅ | nd | 4,4 | 3,6 |
| Température de figeage (°C) | env.10 | env.5 | env. 0 |
| liquide homogène à (°C) | env.20 | env.15 | env.10 |

| | | | |
|---|---|---|---|
| A étant le méthylester de l'acide 2,2'-azobisisobutyrique B étant le méthyl-éthylester de l'acide 2,2'-azobisisobutyrique C étant l'éthylester de l'acide 2,2'-azobisisobutyrique nd signifiant non déterminé. | | | |

Le rapport molaire est calculé en divisant le nombre total de groupes -COOCH3 par le nombre total de groupes -COOC2H5 présents dans le mélange final.

A titre comparatif des mélanges des produits A (solide de point de fusion de 26 à 29°C) et C (liquide jusqu'à -20°C) préparés séparément ne donnent pas des liquides à température ambiante dans les mêmes rapports molaires -COOCH₃/-COOC₂H₅ mais des mélanges solide/liquide et figent rapidement par refroidissement à 15°C.

En faisant varier le rapport des alcools entre eux, on fait varier la composition des mélanges. Ainsi en travaillant comme décrit ci-dessus mais avec 4,3 moles d'acide chlorhydrique et avec en alcool:
a) méthanol 1,2 moles = 38,4g + éthanol 1,2 mole = 55,2g;
b) méthanol 1,6 moles = 51,2g + éthanol 0,8 mole = 36,8g;
c) méthanol 2,0 moles = 64g + éthanol 0,4 mole = 18,4g

0n obtient les résultats suivants, avec A, B et C ayant les significations ci-dessus.

**TABLEAU 3**

| Composition, % molaire | a | b | c |
|---|---|---|---|
| A | 40,0 | 61,0 | 81,0 |
| B | 38,0 | 30,0 | 15,0 |
| C | 18,5 | 6,0 | 1,0 |
| Divers | 3,5 | 3,0 | 3,0 |
| Rapport molaire | | | |
| -COOCH₃/-COOC₂H₅ | 1,5 | 3,6 | 10,5 |
| Température de figeage (C°) | env.-10 | env.0 | env.15 |
| Liquide homogène à (°C) | env.5 | env.10 | env.20 |

A titre comparatif, un produit préparé par mélange de A et C dans un rapport molaire de 1,5 donne un mélange solide/liquide dès environ 15°C et dans un rapport molaire 3,6 fige à environ 15°C.

### Exemple 6

a) A un mélange de 600g de toluène, 164g de 2,2'-azobisisobutyronitrile et 36,8g d'éthanol (0,8 mole), on ajoute en refroidissant à 15-20°C en 2 heures 64,2g d'acide chlorhydrique gazeux (1,76 moles). On maintient le mélange sous agitation pendant 4 heures à 20°C. On ajout ensuite 51,2g de méthanol (1,6 moles) puis en refroidissant à 15-20°C on ajoute en 2 heures 30' 92,7g d'acide chlorhydrique gazeux (2,54 moles). On maintient le mélange réactionnel sous agitation pendant 15 heures à environ 15°C. On coule la suspension obtenue dans 600g d'eau préchauffée à environ 25°C sans dépasser 30°C. On agite environ 1 heure à 30°C. On élimine le toluène par entraînement azéotropique sous vide à une température d'environ 35°C. On décante la phase organique supérieure.
b) On réalise dans les mêmes conditions réactionnelles la réaction suivante: ajout de 55,2g d'éthanol (1,2 moles) et 96,4g d'acide chlorhydrique gazeux (2,64 moles) dans un premier temps puis de 38,4g de méthanol (1,2 moles) et de 60,6g d'acide chlorhydrique gazeux (1,66 moles) dans un deuxième temps.

Les caractéristiques physiques des compositions ainsi obtenues sont consignées dans le tableau 4 ci-dessous.

**TABLEAU 4**

| Exemple 6 | (a) | (b) |
|---|---|---|
| Température de figeage (°C) | 0 | -20 |
| Liquide homogène à (°C) | 5 | -5 |

Par cet ajout d'alcool le plus lourd en premier, on constate un abaissement de la température de figeage et de celle d'obtention d'un liquide homogène (voir la comparaison avec l'exemple 5, a et b).

## Revendications

1. Un procédé de préparation d'un chlorhydrate d'azoiminoéther comprenant la réaction d'un azonitrile entre 15 et 25°C, pendant une durée de 8 à 24 heures, avec un alcool choisi parmi l'éthanol ou un alcool supérieur ou un mélange d'alcools et de l'acide chlorhydrique dans un solvant aromatique, dans lequel le rapport molaire R = HCl/azonitrile est compris entre 4 et 6.

2. Le procédé selon la revendication 1 dans lequel l'azonitrile est formé *in situ* par réaction de l'hydrazonitrile correspondant avec du chlore.

3. Le procédé selon la revendication 1 ou 2 dans lequel le solvant aromatique est sélectionné dans le groupe consistant en toluène, chlorobenzène, xylène, benzène.

4. Le procédé selon la revendication 2 dans lequel le solvant est le chlorobenzène.

5. Le procédé selon l'une quelconque des revendications 1 à 4 dans lequel l'alcool est l'éthanol.

6. Le procédé selon l'une quelconque des revendications 1 à 4 dans lequel l'alcool utilisé est constitué d'un mélange d'alcools.

7. Le procédé selon la revendication 6 dans lequel le mélange comprend du méthanol.

8. Le procédé selon la revendication 6 ou 7 dans lequel le mélange comprend du méthanol et de l'éthanol.

9. Le procédé de préparation selon l'une quelconque des revendications 1 à 8 dans lequel le chlorhydrate d'azoiminoéther répond à la formule (II) 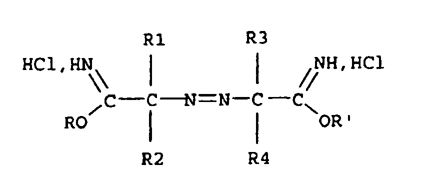 dans laquelle:
R1, R2, R3 et R4, identiques ou différents, sont sélectionnés indépendamment dans le groupe consistant en:
alkyles linéaires ou ramifiés en C1-C9 (de préférence C1-C4) non substitués, ou substitués par un ou plusieurs substituants sélectionnés parmi les substituants hydroxyle, alkoxy en C1-C6, halogène;
cycloalkyles en C3-C6, non substitués ou substitués par un ou plusieurs substituants sélectionnés parmi alkyle en C1-C6, alkoxy en C1-C6, hydroxy, halogène;
aralkyles en C7-C12, non substitués ou substitués par un ou plusieurs substituants sélectionnés parmi alkyle en C1-C6, alkoxy en C1-C6, hydroxy, halogène;
aryles en C7-C12 non substitués ou substitués par un ou plusieurs substituants sélectionnés parmi alkyle en C1-C6, alkoxy en C1-C6, hydroxy, halogène;
au moins une des combinaisons R1-R2 et R3-R4 pouvant éventuellement former un cycle aliphatique,
R et R', identiques ou différents, sont sélectionnés indépendamment dans le groupe consistant en radicaux aliphatiques linéaires ou ramifiés en C1-C10, de préférence en C1-C4.

10. Le procédé de préparation selon la revendication 9 dans lequel dans la formule (II), R et R' sont différents l'un de l'autre et sont sélectionnés parmi les radicaux aliphatiques linéaires en C1-C4.

11. Le procédé de préparation selon la revendication 9 ou 10 dans lequel dans la formule (II), R1, R2, R3 et R4 sont des groupes alkyles en C1-C4.

12. Un procédé de préparation d'un ester d'acide azocarboxylique comprenant la synthèse d'un chlorhydrate d'azoiminoéther par le procédé défini dans l'une quelconque des revendications 1 à 11, et l'hydrolyse en présence d'eau du chlorhydrate d'azoiminoéther ainsi obtenu.

13. Le procédé de préparation selon la revendication 12 dans lequel l'hydrolyse est mise en oeuvre par addition successive d'eau au mélange réactionnel ou par coulée du mélange réactionnel dans de l'eau, à une température comprise entre 15°C et 50°C, de préférence entre 2°C et 35°C.

14. Le procédé de préparation selon la revendication 12 dans lequel après la synthèse, le chlorhydrate d'azoiminoéther est séparé par filtration, lavé avec un solvant organique et hydrolysé par addition progressive du gâteau de filtration dans de l'eau à une température comprise entre 15°C et 50°C, de préférence entre 25°C et 35°C.

15. Un procédé de préparation d'une composition liquide d'esters d'acides azocarboxyliques comprenant la synthèse de chlorhydrate d'azoiminoéthers selon la revendication 6, 7, 8, 9 ou 10, l'hydrolyse des sels ainsi obtenus en présence d'eau et l'isolement de la phase organique contenant les esters.

16. Le procédé de préparation selon la revendication 15 dans lequel on fait réagir dans un premier temps l'alcool le plus lourd puis dans un deuxième temps l'alcool le moins lourd.

17. Un procédé de préparation d'initiateurs de polymérisation comprenant la synthèse d'un ester d'acide azocarboxylique par le procédé selon l'une quelconque des revendications 12 à 16 et le cas échéant la transformation de cet ester en initiateur par des procédés connus.

18. Un procédé de préparation d'un dérivé azoguanyl comprenant la préparation d'un chlorhydrate d'azoiminoéther par le procédé selon l'une quelconque des revendications 1 à 11 et la réaction de ce dernier avec de l'ammoniac ou une amine en présence d'alcool.

## Claims

1. A process for the preparation of an azoiminoether hydrochloride comprising the reaction of an azonitrile between 15 and 25°C, for a period of time of 8 to 24 hours, with an alcohol chosen from ethanol a higher alcohol or a mixture of alcohols and hydrochloric acid in an aromatic solvent, wherein the molar ratio R = HCI/azonitrile is between 4 and 6.

2. The process as claimed in claim 1, in which the azonitrile is formed *in situ* by reaction of the corresponding hydrazonitrile with chlorine.

3. The process as claimed in claim 1 or 2, in which the solvent is selected from the group consisting of toluene, chlorobenzene, xylene and benzene.

4. The process as claimed in claim 2, in which the solvent is chlorobenzene.

5. The process as claimed in any one of claims I to 4, in which the alcohol is ethanol.

6. The process as claimed in any one of claims 1 to 4, in which the alcohol used is composed of a mixture of alcohols.

7. The process as claimed in claim 6, in which the mixture comprises methanol.

8. The process as claimed in claim 6 or 7, in which the mixture comprises methanol and ethanol.

9. The preparation process as claimed in any one of claims 1 to 8, in which the azoiminoether hydrochloride corresponds to the formula (II) 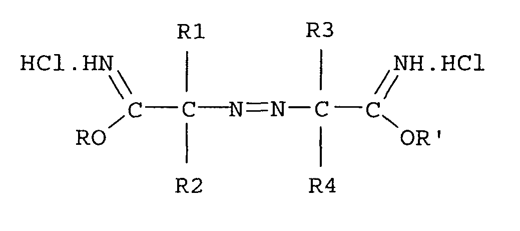 in which:
R1, R2, R3 and R4, which are identical or different, are independently selected from the group consisting of: linear or branched C1-C9 (preferably C1-C4) alkyls which are unsubstituted or substituted by one or more substituents selected from hydroxyl, C1-C6 alkoxy or halogen substituents;
C3-C6 cycloalkyls which are unsubstituted or substituted by one or more substituents selected from C1-C6 alkyl, C1-C6 alkoxy, hydroxyl or halogen;
C7-C12 aralkyls which are unsubstituted or substituted by one or more substituents selected from C1-C6 alkyl, C1-C6 alkoxy, hydroxyl or halogen;
C7-C12 aryls which are unsubstituted or substituted by one or more substituents selected from C1-C6 alkyl, C1-C6 alkoxy, hydroxyl or halogen;
it being possible for at least one of the R1-R2 and R3-R4 combinations optionally to form an aliphatic ring,
R and R', which are identical or different, are independently selected from the group consisting of linear or branched C1-C10, preferably C1-C4, aliphatic radicals.

10. The preparation process as claimed in claim 9, in which, in the formula (II), R and R' are different from one another and are selected from linear C1-C4 aliphatic radicals.

11. The preparation process as claimed in claim 9 or 10, in which, in the formula (II), R1, R2, R3 and R4 are C1-C4 alkyl groups.

12. A process for the preparation of an azocarboxylic acid ester comprising the synthesis of an azoiminoether hydrochloride by the process defined in any one of claims 1 to 11 and the hydrolysis in the presence of water of the azoiminoether hydrochloride thus obtained.

13. The preparation process as claimed in claim 12, in which the hydrolysis is carried out by successive addition of water to the reaction mixture or by running the reaction mixture into water, at a temperature of between 15°C and 50°C, preferably between 2°C and 35°C.

14. The preparation process as claimed in claim 12, in which, after the synthesis, the azoiminoether hydrochloride is separated by filtration, washed with an organic solvent and hydrolyzed by gradual addition of the filtration cake to water at a temperature of between 15°C and 50°C, preferably between 25°C and 35°C.

15. A process for the preparation of a liquid composition of azocarboxylic acid esters comprising the synthesis of azoiminoether hydrochloride as claimed in claim 6, 7, 8, 9 or 10, the hydrolysis of the salts thus obtained in the presence of water and the isolation of the organic phase comprising the esters.

16. The preparation process as claimed in claim 15, in which the heaviest alcohol is reacted in a first step and then the lightest alcohol is reacted in a second step.

17. A process for the preparation of polymerization initiators comprising the synthesis of an azocarboxylic acid ester by the process as claimed in any one of claims 12 to 16 and, if appropriate, the conversion of this ester to an initiator by known processes.

18. A process for the preparation of an azoguanyl derivative comprising the preparation of an azoiminoether hydrochloride by the process as claimed in any one of claims 1 to 11 and the reaction of the latter with ammonia or an amine in the presence of an alcohol.

## Patentansprüche

1. Verfahren zur Herstellung eines Azoiminoether-Hydrochlorids aufweisend die Reaktion, zwischen 15 und 25°C und während 8 bis 24 Stunden, eines Azonitrils mit einem Alkohol der aus der Gruppe die aus Ethanol, ein höherer Alkohol, einem Gemisch von Alkoholen und Chlorwasserstoffsaure ausgewählt ist, in einem aromatischen Lösungsmittel, bei dem das Molverhältnis R = HCl/Azonitril zwichen 4 und 6 ist;

2. Verfahren nach Anspruch 1, bei dem das Azonitril in situ durch Reaktion des entsprechenden Hydrazonitrils mit Chlor gebildet wird.

3. Verfahren nach Anspruch 1 oder 2, bei dem das Lösungsmittel ausgewahlt wird aus der Gruppe, die besteht aus Toluol, Chlorbenzol, Xylol, Benzol.

4. Verfahren nach Anspruch 2, bei dem das Lösungsmittel Chlorbenzol ist.

5. Verfahren nach einem der Ansprüche 1 bis 4, bei dem der Alkohol Ethanol ist.

6. Verfahren nach einem der Ansprüche 1 bis 4, bei dem der verwendete Alkohol aus einem Gemisch von Alkoholen besteht.

7. Verfahren nach Anspruch 6, bei dem das Gemisch Methanol enthalt.

8. Verfahren nach Anspruch 6 oder 7, bei dem das Gemisch Methanol und Ethanol enthalt.

9. Herstellungsverfahren nach einem der Ansprüche 1 bis 8, bei dem das Azoiminoether-Hydrochlorid der Formel (II) entspricht 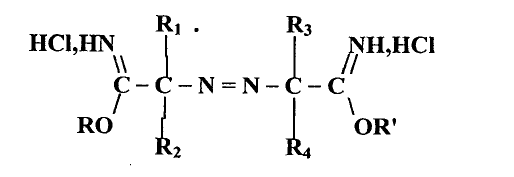 in der
- R₁, R₂, R₃ und R₄, die gleich oder verschieden sind, unabhangig voneinander ausgewahlt sind aus der Gruppe, die besteht aus: linearen oder verzweigten C₁-C₉ (bevorzugt C₁-C₄)-Alkylen, die unsubstituiert oder mit einem oder mehreren Substituenten, die unter den Hydroxyl-, C₁-C₆-Alkoxy-, HalogenSubstituenten ausgewahlt sind, substituiert sind;
- C₃-C₆-Cycloalkylen, die unsubstituiert oder mit einem oder mehreren Substituenten, die ausgewahlt sind aus C₁-C₆-Alkyl, C₁-C₆-Alkoxy, Hydroxy, Halogen, substituiert sind;
- C₇-C₁₂-Aralkylen, die unsubstituiert oder mit einem oder mehreren Substituenten, die ausgewahlt sind aus C₁-C₆-Alkyl, C₁-C₆-Alkoxy, Hydroxy, Halogen, substituiert sind;
- C₇-C₁₂-Arylen, die unsubstituiert oder mit einem oder mehreren Substituenten, die ausgewahlt sind aus C₁-C₆-Alkyl, C₁-C₆-Alkoxy, Hydroxy, Halogen, substituiert sind;
wo bei mindestens eine der Kombinationen R₁-R₂ und R₃-R₄ gegebenenfalls einen aliphatischen Ring bilden kann,
- R und R', die gleich oder verschieden sind, unabhangig voneinander ausgewahlt sind aus der Gruppe, die besteht aus aliphatischen, linearen oder verzweigten C₁-C₁₀-Resten, bevorzugt C₁-C₄-Resten.

10. Herstellungsverfahren nach Anspruch 9, bei dem in der Formel (II) R und R' voneinander verschieden sind und aus den aliphatischen linearen C₁-C₄-Resten ausgewahlt sind.

11. Herstéllungsverfahren nach Anspruch 9 oder 10, bei dem in der Formel (II) R₁, R₂, R₃ und R₄ C₁-C₄-Alkylgruppen sind.

12. Verfahren zur Herstellung eines Azocarbonsaureesters aufweisend die Synthese eines Azoiminoether-Hydrochlorids durch das in einem der Ansprüche 1 bis 11 definierte Verfahren und die Hydrolyse des so erhaltenen Azoiminoether-Hydrochlorids in Anwesenheit von Wasser.

13. Herstellungsverfahren nach Anspruch 12, bei dem die Hydrolyse durch sukzessive Zugabe von Wasser zu dem Reaktionsgemisch oder durch Fließen lassen des Reaktionsgemisch in Wasser bei einer Temperatur zwischen 15°C und 50°C, bevorzugt zwischen 25°C und 35°C, durchgeführt wird.

14. Herstellungsverfahren nach Anspruch 12, bei dem Azoiminoether-Hydrochlorids nach der Synthese durch Filtration abgetrennt, mit einem organischen Lösungsmittel gewaschen und durch fortschreitende Zugabe des Filterkuchens in Wasser bei einer Temperatur zwischen 15°C und 50°C, bevorzugt zwischen 25°C und 35°C, durchgeführt wird.

15. Verfahren zur Herstellungs einer flüssigen Zusammensetzung von Azocarbonsäureestern, aufweisend dis Synthese von Azoiminoether-Hydrochloriden nach Anspruch 6, 7, 8, 9 oder 10, die Hydrolyse der so erhaltenen Salze in Anwesenheit von Wasser und die Isolierung der die Ester enthaltenden organische Phase.

16. Herstellungsverfahren nach Anspruch 15, bei dem man während einer ersten Zeit dem schwerten Alkohol, dann während einer zweiten Zeit den am wenigsten schweren Alkohol reagieren läßt.

17. Verfahren zur Herstellung von Polymerisationsstartern aufweisend die Synthese eines Azocarbonsaureesters nach dem Verfahren gemaß einem der Ansprüche 12 bis 16 und gegebenenfalls die Umwandlung dieses Esters in einen Starter durch bekannte Verfahren.

18. Verfahren zur Herstellung eines Azoguanyl-Derivats, aufweisend die Herstellung eines Azoiminoether-Hydrochlorids durch das Verfahren gemalS einem der Ansprüche 1 bis 11 und die Reaktion dieses Letzteren mit Ammoniak oder einem Amin in Anwesenheit von Alkohol.
